# EUROPEAN PATENT APPLICATION

(11) **EP 4 763 039 A1**
(43) Date of publication of application: **24.06.2026**
(21) Application number: 25220801.2
(22) Date of filing: 04.12.2025
(51) Int. Cl.: A61B 1/00, A61B 1/24, A61C 9/00

(54) **WIRELESS INTRAORAL SCAN IMAGE FORMING APPARATUS**

(30) Priority: 18.12.2024 KR 20240189588
(71) Applicant: Huvitz Co., Ltd., Anyang-si, Gyeonggi-do 14055 (KR)
(72) Inventor: CHO, Min Soo, 14055 Anyang-si (KR); LEE, Weon Joon, 14055 Anyang-si (KR)
(74) Representative: Henkel & Partner mbB

(57) **Abstract**

A wireless intraoral scan image forming apparatus that wirelessly acquires three-dimensional shape information of intraoral structures is disclosed. The wireless intraoral scan image forming apparatus includes: a wireless intraoral scanner (10) configured to irradiate one measurement light having a predetermined pattern onto intraoral structures, obtain two-dimensional shape image data of the intraoral structures from one two-dimensional reflected light image reflected from the intraoral structures, convert it into a wireless signal, and transmit the wireless signal; a data processor (22) configured to output a control signal for driving the wireless intraoral scanner (10) and form a three-dimensional shape image of the intraoral structures from the two-dimensional shape image data of the intraoral structures obtained from the wireless intraoral scanner (10); and a wireless transmission/reception device (80) configured to receive the wireless signal transmitted from the wireless intraoral scanner (10), convert the wireless signal into a wired signal and transmit the wired signal to the data processor (22), convert a wired signal transmitted from the data processor (22) into a wireless signal and transmit the wireless signal to the wireless intraoral scanner (10).

## Description

### CROSS-REFERENCE TO RELATED APPLICATION

This application claims benefit of priority to Korean Patent Application No. 10-2024-0189588 filed on December 18, 2024, the entire contents of which are incorporated herein by reference.

### FIELD OF THE DISCLOSURE

The present invention relates to an intraoral scan image forming apparatus, and more particularly, to a wireless intraoral scan image forming apparatus that wirelessly acquires three-dimensional shape information of intraoral structures.

### BACKGROUND

In general, at dental clinics and the like, the shape of a patient's teeth or gums in the oral cavity is examined, and based on this, the patient's oral condition is diagnosed or prosthetics are manufactured. Recently, optical intraoral scan image forming apparatuses have been used that optically scan and capture the interior of a patient's oral cavity without physical contact to obtain shape images of intraoral structures.

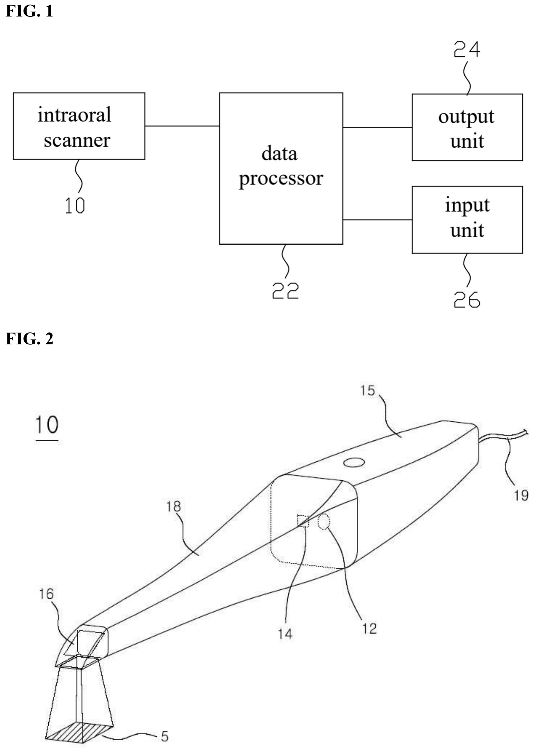

FIG. 1 is a diagram illustrating the configuration of a conventional intraoral scan image forming apparatus. As shown in FIG. 1, a conventional intraoral scan image forming apparatus includes an intraoral scanner 10, a data processor 22, an output unit 24, and an input unit 26.

The intraoral scanner 10 is a device that optically scans and captures a patient's intraoral structures, such as teeth and gums, to obtain two-dimensional shape image data of the intraoral structures. FIG. 2 is a perspective view for explaining the configuration of a conventional intraoral scanner 10. As shown in FIG. 2, the conventional intraoral scanner 10 includes a measurement light source 12 that irradiates measurement light onto the intraoral structures, a shape detector 14 that detects reflected light formed by the reflection of the measurement light from the intraoral structures, an intraoral scanner body 15 that accommodates the measurement light source 12 and the shape detector 14, and an intraoral tip 18. The intraoral tip 18 is equipped with a reflection mirror 16 that reflects the measurement light generated from the measurement light source 12 to irradiate it onto the intraoral structures and reflects the reflected light from the intraoral structures to guide it to the shape detector 14. In FIG. 2, reference numeral 19 denotes a wired signal line that supplies power to the intraoral scanner 10 and transmits data and/or control signals between the intraoral scanner 10 and the data processor 22.

A user holds the intraoral scanner body 15 by hand, positions the intraoral tip 18 inside the patient's oral cavity, irradiates measurement light onto a predetermined area 5 inside the oral cavity using the measurement light source 12, and detects the reflected light generated by the reflection of the measurement light from the area 5 using the shape detector 14 to obtain two-dimensional shape image data of the intraoral structures.

Referring back to FIG. 1, the data processor 22 processes the two-dimensional shape image data of the intraoral structures obtained from the intraoral scanner 10 to generate a three-dimensional shape image of the intraoral structures necessary for the patient's examination or treatment. The output unit 24 is a device that displays on a screen the processing of the intraoral scan image performed by the data processor 22, the shape image of the intraoral structures obtained thereby, and the like. The input unit 26 is a device for inputting user commands to the data processor 22.

Conventionally, since the intraoral scanner 10 that scans the shape of the intraoral structures and the data processor 22 that processes the shape image data obtained from the intraoral scanner 10 are connected by the wired signal line 19, there is inconvenience in that the user cannot freely use the intraoral scanner 10.

To improve such disadvantages, wireless intraoral scanners have also been used, in which a wireless data transmission/reception device such as Bluetooth or Wi-Fi and a power source for driving the intraoral scanner 10, i.e., a battery, are mounted inside the intraoral scanner 10, and communication between the intraoral scanner 10 and the data processor 22 is performed wirelessly.

In a conventional wireless intraoral scanner, when scanning one measurement area 5, a plurality of measurement lights having patterns that change over time, i.e., time-varying patterns, are sequentially irradiated onto the measurement area 5, and reflected light corresponding to each measurement light having a time-varying pattern is detected to generate a plurality of two-dimensional shape image data for one measurement area 5. The plurality of two-dimensional shape image data thus generated is wirelessly transmitted to the data processor 22. The data processor 22 processes and synthesizes the transmitted data to generate (reconstruct) a three-dimensional shape image of the measurement area 5.

In the conventional wireless intraoral scanner, since a plurality of two-dimensional shape image data is wirelessly transmitted, a lot of time is required for data transmission, making real-time data transmission difficult, and also the position of the measurement area 5 may change during the scanning process, so there is a disadvantage that the scanning operation of the intraoral structures is difficult to perform smoothly. In addition, since the wireless intraoral scanner must process a plurality of two-dimensional shape image data, there is a need for higher performance of the processor mounted in the wireless intraoral scanner, and there is a disadvantage that the power consumption in the wireless intraoral scanner increases.

[Prior Art Documents]
(Patent Document 1) U.S. Patent No. 9,629,551
(Patent Document 2) U.S. Patent No. 10,064,553
(Patent Document 3) U.S. Patent No. 10,695,151
(Patent Document 4) U.S. Patent No. 11,076,146
(Non-Patent Document 1) Structured-light 3D surface imaging: a tutorial, Jason Geng, Advances in Optics and Photonics 3, 128 - 160 (2011) doi:10.1364/AOP.3.000128

### SUMMARY OF THE DISCLOSURE

### TECHNICAL OBJECTS

An object of the present invention is to provide a wireless intraoral scan image forming apparatus capable of reducing the size of two-dimensional shape image data, thereby reducing the wireless transmission time of the data and improving the convenience of intraoral structure scanning operations.

Another object of the present invention is to provide a wireless intraoral scan image forming apparatus capable of reducing the computational burden and power consumption of a wireless intraoral scanner.

Yet another object of the present invention is to provide a wireless intraoral scan image forming apparatus capable of accurately acquiring the three-dimensional shape and color of intraoral structures.

### TECHNICAL SOLUTION

To achieve the above objects, the present invention provides a wireless intraoral scan image forming apparatus comprising: a wireless intraoral scanner configured to irradiate one measurement light having a predetermined pattern onto intraoral structures, obtain two-dimensional shape image data of the intraoral structures from one two-dimensional reflected light image reflected from the intraoral structures, convert it into a wireless signal, and transmit the wireless signal; a data processor configured to output a control signal for driving the wireless intraoral scanner and form a three-dimensional shape image of the intraoral structures from the two-dimensional shape image data of the intraoral structures obtained from the wireless intraoral scanner; and a wireless transmission/reception device configured to receive the wireless signal transmitted from the wireless intraoral scanner, convert the wireless signal into a wired signal and transmit the wired signal to the data processor, convert a wired signal transmitted from the data processor into a wireless signal and transmit the wireless signal to the wireless intraoral scanner.

### EFFECTS OF THE DISCLOSURE

The wireless intraoral scan image forming apparatus according to the present invention can reduce the size of two-dimensional shape image data, thereby reducing the wireless transmission time of the data, improving the convenience of intraoral structure scanning operations, and reducing the computational burden and power consumption of the wireless intraoral scanner.

### BRIEF DESCRIPTION OF THE DRAWINGS

FIG. 1 is a diagram illustrating the configuration of a conventional intraoral scan image forming apparatus.
FIG. 2 is a perspective view for explaining the structure of a conventional intraoral scanner.
FIG. 3 is a diagram illustrating the configuration of a wireless intraoral scan image forming apparatus according to an embodiment of the present invention.
FIG. 4 is a diagram illustrating the configuration of a wireless intraoral scanner that can be used in the present invention.
FIG. 5 is a diagram illustrating an example of measurement light having a color pattern obtained by combining three or more colors in three or more arrangements.
FIG. 6 is photographs showing states in which a color pattern and white illumination light generated from a measurement light source of an intraoral scanner are irradiated onto a measurement area of intraoral structures, respectively.
FIG. 7 is a diagram illustrating the configuration of a wireless transmission/reception device that can be used in the present invention.
FIG. 8 is a diagram illustrating the configuration of a wireless intraoral scan image forming apparatus according to another embodiment of the present invention.

### DETAILED DESCRIPTION OF THE PREFERRED EMBODIMENTS

Hereinafter, the present invention will be described in detail with reference to the accompanying drawings. In the accompanying drawings, the same reference numerals are assigned to components that perform the same or similar functions as in the prior art.

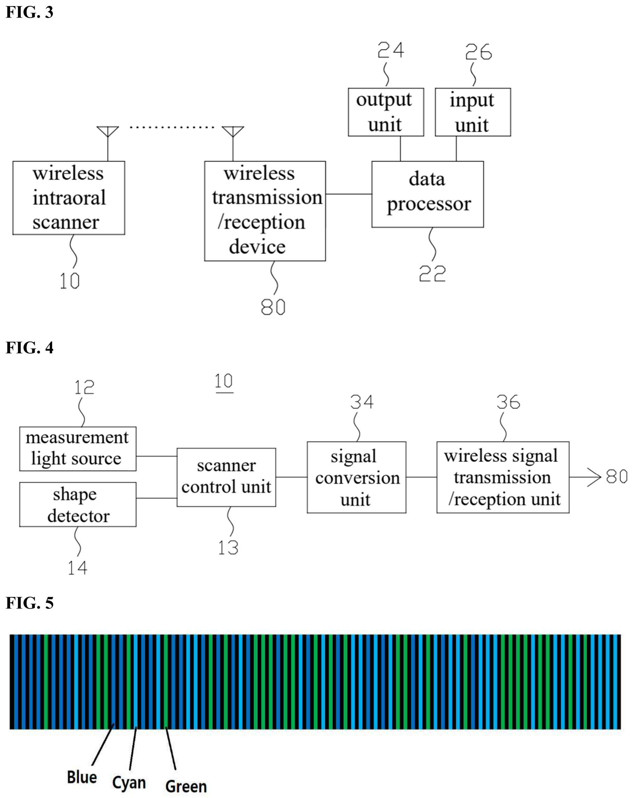

FIG. 3 is a diagram illustrating the configuration of a wireless intraoral scan image forming apparatus according to an embodiment of the present invention. As shown in FIG. 3, the wireless intraoral scan image forming apparatus according to the present invention includes a wireless intraoral scanner 10, a wireless transmission/reception device 80, and a data processor 22, and may further include an output unit 24 and an input unit 26 as needed.

The intraoral scanner 10 irradiates one measurement light having a predetermined pattern onto intraoral structures, such as teeth and gums, obtains two-dimensional shape image data of the intraoral structures from one two-dimensional (2D) reflected light image reflected from the intraoral structures, converts it into a wireless signal, and transmits the wireless signal to the data processor 22. In addition, the intraoral scanner 10 receives a wireless control signal transmitted wirelessly from the data processor 22 and scans the intraoral structures according to the received wireless signal.

FIG. 4 is a block diagram illustrating the configuration of a wireless intraoral scanner 10 that can be used in the present invention. Referring to FIGS. 2 and 4, the wireless intraoral scanner 10 that can be preferably used in the present invention includes: (a) a measurement light source 12 configured to irradiate measurement light onto the intraoral structures; (b) a shape detector 14 configured to detect reflected light formed by the reflection of the measurement light from the intraoral structures; (c) a scanner control unit 13 configured to output a control signal for controlling the measurement light source 12 and obtain two-dimensional shape image data of the intraoral structures from the signal of the reflected light detected by the shape detector 14; (d) a signal conversion unit 34 configured to convert the two-dimensional shape image data transmitted from the scanner control unit 13 into a wireless signal and convert the control signal transmitted as a wireless signal from the wireless transmission/reception device 80 into a wired signal and transmit the wired signal to the scanner control unit 13; and (e) a wireless signal transmission/reception unit 36 configured to wirelessly transmit the two-dimensional shape image data in the form of the wireless signal converted by the signal conversion unit 34 to the wireless transmission/reception device 80 and receive the control signal transmitted as a wireless signal from the wireless transmission/reception device 80.

In addition, as shown in FIG. 2, the wireless intraoral scanner 10 may include an intraoral scanner body 15 that accommodates the measurement light source 12 and the shape detector 14, and an intraoral tip 18 equipped with a reflection mirror 16. The reflection mirror 16 reflects the measurement light generated from the measurement light source 12 to irradiate it onto the intraoral structures and also reflects the reflected light from the intraoral structures to guide the reflected light to the shape detector 14.

A user holds the intraoral scanner body 15 by hand, positions the intraoral tip 18 inside the patient's oral cavity, irradiates measurement light onto a predetermined area 5 inside the oral cavity using the measurement light source 12, and detects the reflected light generated by the reflection of the measurement light from the area 5 using the shape detector 14 to obtain two-dimensional shape image data of the intraoral structures.

In the present invention, the measurement light generated from the measurement light source 12 is structured light having a predetermined pattern. In the present invention, one measurement light having a predetermined pattern, i.e., one pattern, is irradiated onto intraoral structures having a three-dimensional shape, one two-dimensional (2D) reflected light image reflected from the intraoral structures is obtained, and then analyzed to form a three-dimensional shape image of the intraoral structures (3D shape image reconstruction).

The pattern of the measurement light used in the present invention may be a color pattern or a black-and-white pattern. In the color pattern, when k colors are continuously arranged in n sequences, k^n different de Bruijn sequences, i.e., color combinations, can be obtained, and a color-coded pattern can be formed by combining them. FIG. 5 is a diagram illustrating an example of measurement light having a color pattern obtained by arranging three or more colors, for example, 3 to 5 colors, in three or more, for example, 3 to 6 arrangements. As shown in FIG. 5, when the colors are three colors: Blue, Green, Cyan (k=3), and three colors are arranged in four sequences (n=4), the number of available combinations is 3^4, i.e., 81.

The black-and-white pattern may be a monochromatic pattern obtained by combining dots, lines, and spaces. A method of acquiring a three-dimensional shape image of an object using such measurement light having one color pattern or black-and-white pattern can be performed, for example, by the method disclosed in Structured-light 3D surface imaging: a tutorial, Jason Geng, Advances in Optics and Photonics 3, 128 - 160 (2011) doi:10.1364/AOP.3.000128.

The measurement light of the color pattern or black-and-white pattern thus generated is irradiated onto a target, i.e., a predetermined area 5 of the intraoral structures, and then the reflected light from the intraoral structures, i.e., from the target, is detected by the shape detector 14, i.e., an internal camera sensor. Specifically, using the scanner control unit 13 of the intraoral scanner 10, a two-dimensional image (2D image) of the target is obtained from the signal of the shape detector 14, i.e., the camera sensor, in accordance with a predetermined synchronization signal. Since the shape of the reflected light obtained from the intraoral scanner 10, i.e., the two-dimensional reflected image, changes according to the three-dimensional shape of the intraoral structures, by analyzing this, a three-dimensional shape image of the intraoral structures can be obtained (i.e., reconstructed).

In addition, as needed, in the wireless intraoral scanner 10, the measurement light source 12 may irradiate illumination light, for example, white illumination light, onto the intraoral structures, the shape detector 14 may obtain a two-dimensional reflected color image formed by the reflection of the illumination light from the intraoral structures. The data processor 22 may obtain color information of the intraoral structures from the two-dimensional reflected color image. More specifically, the scanner control unit 13 may obtain two-dimensional reflected color image data from the two-dimensional reflected color image, and the data processor 22 may obtain a color image, i.e., color information, of the intraoral structures from the two-dimensional reflected color image data.

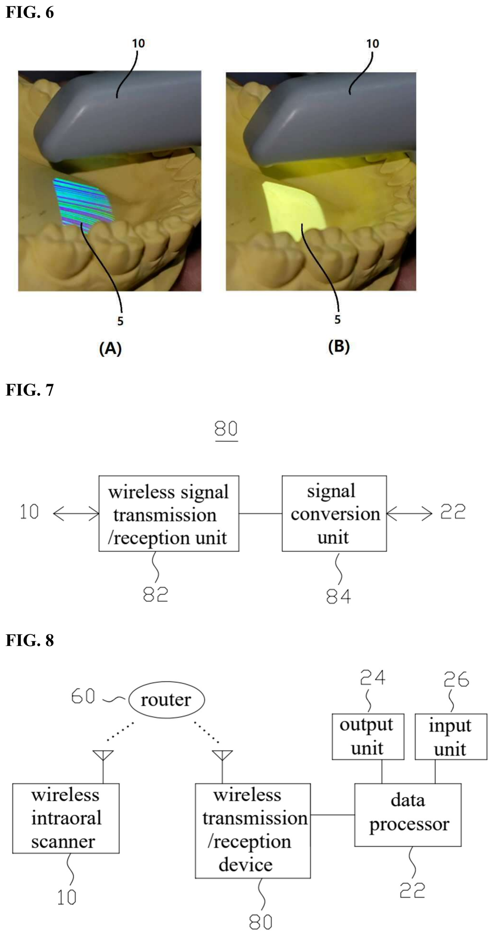

FIG. 6 is photographs showing states in which a color pattern measurement light (A in FIG. 6) and white illumination light (B in FIG. 6) generated from the measurement light source 12 of the intraoral scanner 10 are irradiated onto the measurement area 5 of the intraoral structures, respectively. By integrating the color image of the intraoral structures into the three-dimensional shape image of the intraoral structures, a color image of the three-dimensional shape can be obtained.

The two-dimensional shape image data obtained from the scanner control unit 13 of the intraoral scanner 10 is, if necessary, together with the two-dimensional color image data, converted into a wireless signal by the signal conversion unit 34, and wirelessly transmitted to the data processor 22 by the wireless signal transmission/reception unit 36. The intraoral scanner 10 is equipped with a power source (not shown), for example, a rechargeable battery, for driving the intraoral scanner 10.

The data processor 22 outputs the control signal for driving the wireless intraoral scanner 10 and forms a three-dimensional shape image of the intraoral structures necessary for the patient's examination or treatment from the two-dimensional shape image data of the intraoral structures obtained from the intraoral scanner 10. The shape of the two-dimensional image obtained from the intraoral scanner 10 changes according to the three-dimensional shape of the intraoral structures. Therefore, the data processor 22 can analyze (convert) the obtained two-dimensional image to obtain the three-dimensional shape image of the intraoral structures. In addition, as needed, the data processor 22 can analyze the color of the two-dimensional reflected image generated by the reflection of illumination light, for example, white illumination light, from the intraoral structures to obtain a color image, i.e., color information, of the intraoral structures. The data processor 22 may be a computer equipped with application software (S/W) capable of processing shape image data and/or color image data (i.e., performing analysis, movement, comparison, registration, etc. of shape and/or color image data), for example, a personal computer (PC) or a notebook computer.

The wireless transmission/reception device 80 receives the wireless signal transmitted from the wireless intraoral scanner 10, converts the wireless signal into a wired signal and transmits the wired signal to the data processor 22. The wireless transmission/reception device 80 also converts a wired control signal transmitted from the data processor 22 into a wireless control signal and transmits it to the intraoral scanner 10.

FIG. 7 is a block diagram illustrating the configuration of a wireless transmission/reception device 80 that can be used in the present invention. As shown in FIG. 7, the wireless transmission/reception device 80 may include: (a) a wireless signal transmission/reception unit 82 configured to receive two-dimensional shape image data transmitted wirelessly from the intraoral scanner 10 and also wirelessly transmit the control signal transmitted from the data processor 22 to the intraoral scanner 10; and (b) a signal conversion unit 84 configured to convert the two-dimensional shape image data transmitted from the wireless signal transmission/reception unit 82 into a wired signal and transmit it to the data processor 22, and also convert the control signal transmitted as a wired signal from the data processor 22 into a wireless signal.

The communication between the wireless intraoral scanner 10 and the wireless transmission/reception device 80 may be performed using a conventional wireless communication protocol such as Bluetooth or Wi-Fi.

Referring back to FIG. 3, the output unit 24 is a device that displays on a screen the processing of the intraoral scan image performed by the data processor 22, the three-dimensional shape image of the intraoral structures obtained thereby, and the like. The output unit 24 is, for example, a conventional display device such as a liquid crystal display (LCD) device.

The input unit 26 is a device for inputting user commands. The input unit 26 is, for example, a device for inputting user operation commands such as movement or rotation of the shape image of the intraoral structures to a user interface displayed on the output unit 24. The input unit 26 is, for example, a conventional input device such as a keyboard or mouse.

FIG. 8 is a diagram illustrating the configuration of a wireless intraoral scan image forming apparatus according to another embodiment of the present invention. The wireless intraoral scan image forming apparatus shown in FIG. 8 differs from the wireless intraoral scan image forming apparatus shown in FIG. 3 in that the wireless communication between the wireless intraoral scanner 10 and the wireless transmission/reception device 80 is not made directly but performed through a router 60.

As shown in FIG. 8, when wireless communication is performed through a general-purpose wireless router 60, the physical distance between the wireless intraoral scanner 10 and "the wireless transmission/reception device 80 and the data processor 22" can be increased. For example, "the wireless transmission/reception device 80 and the data processor 22" may be installed indoors in a hospital or the like, and a user may examine a patient outdoors using the wireless intraoral scanner 10.

The router 60 may be a general-purpose Wi-Fi router, and the wireless communication between the wireless intraoral scanner 10 and the wireless transmission/reception device 80 may be performed through a Wi-Fi communication network.

According to the present invention, by using measurement light having one pattern to obtain one two-dimensional (2D) reflected light image and then analyzing it to form a three-dimensional shape image of the intraoral structures, the transmission bandwidth of wireless data can be significantly reduced. Therefore, according to the present invention, a three-dimensional shape image of the intraoral structures can be smoothly obtained using a general wireless communication method such as Wi-Fi.

In addition, by forming a three-dimensional shape image using one two-dimensional reflected light image having a predetermined pattern instead of a plurality of two-dimensional reflected light images having time-varying patterns, artifacts due to motion of the intraoral structures can be reduced, thereby reducing errors in forming the three-dimensional shape image.

Although the present invention has been described with reference to the accompanying drawings and exemplary embodiments, the present invention is not limited to the contents shown in the drawings and the above-described embodiments. For the purpose of understanding, reference numerals are indicated in the following claims, but the scope of the following claims is not limited to the reference numerals and the contents shown in the drawings, and should be interpreted to encompass all modifications, equivalents, and functions of the exemplary embodiments.

## Claims

1. A wireless intraoral scan image forming apparatus comprising:
a wireless intraoral scanner (10) configured to irradiate one measurement light having a predetermined pattern onto intraoral structures, obtain two-dimensional shape image data of the intraoral structures from one two-dimensional reflected light image reflected from the intraoral structures, convert it into a wireless signal, and transmit the wireless signal;
a data processor (22) configured to output a control signal for driving the wireless intraoral scanner (10) and form a three-dimensional shape image of the intraoral structures from the two-dimensional shape image data of the intraoral structures obtained from the wireless intraoral scanner (10); and
a wireless transmission/reception device (80) configured to receive the wireless signal transmitted from the wireless intraoral scanner (10), convert the wireless signal into a wired signal and transmit the wired signal to the data processor (22), convert a wired signal transmitted from the data processor (22) into a wireless signal and transmit the wireless signal to the wireless intraoral scanner (10).

2. The wireless intraoral scan image forming apparatus of claim 1, wherein the pattern of the measurement light is a color pattern obtained by combining three or more colors in three or more arrangements.

3. The wireless intraoral scan image forming apparatus of claim 1, wherein the pattern of the measurement light is a monochromatic pattern obtained by combining dots, lines, and spaces.

4. The wireless intraoral scan image forming apparatus of claim 1, wherein the wireless intraoral scanner (10) is configured to irradiate illumination light onto the intraoral structures and obtain a two-dimensional reflected color image formed by the reflection of the illumination light from the intraoral structures, and the data processor (22) is configured to further obtain color information of the intraoral structures from the two-dimensional reflected color image.

5. The wireless intraoral scan image forming apparatus of claim 1, wherein the wireless intraoral scanner (10) comprises:
(a) a measurement light source (12) configured to irradiate measurement light onto the intraoral structures;
(b) a shape detector (14) configured to detect reflected light formed by the reflection of the measurement light from the intraoral structures;
(c) a scanner control unit (13) configured to output a control signal for controlling the measurement light source (12) and obtain two-dimensional shape image data of the intraoral structures from the signal of the reflected light detected by the shape detector (14);
(d) a signal conversion unit (34) configured to convert the two-dimensional shape image data transmitted from the scanner control unit (13) into a wireless signal and convert the control signal transmitted as a wireless signal from the wireless transmission/reception device (80) into a wired signal and transmit the wired signal to the scanner control unit (13); and
(e) a wireless signal transmission/reception unit (36) configured to wirelessly transmit the two-dimensional shape image data in the form of the wireless signal converted by the signal conversion unit (34) to the wireless transmission/reception device (80) and receive the control signal transmitted as the wireless signal from the wireless transmission/reception device (80).

6. The wireless intraoral scan image forming apparatus of claim 1, wherein the wireless transmission/reception device (80) comprises:
(a) a wireless signal transmission/reception unit (82) configured to receive two-dimensional shape image data transmitted wirelessly from the intraoral scanner (10) and wirelessly transmit the control signal transmitted from the data processor (22) to the intraoral scanner (10); and
(b) a signal conversion unit (84) configured to convert the two-dimensional shape image data transmitted from the wireless signal transmission/reception unit (82) into a wired signal and transmit the wired signal to the data processor (22), and convert the control signal transmitted as a wired signal from the data processor (22) into a wireless signal.

7. The wireless intraoral scan image forming apparatus of claim 1, wherein a wireless communication between the wireless intraoral scanner (10) and the wireless transmission/reception device (80) is performed through a router (60).

8. The wireless intraoral scan image forming apparatus of claim 1, wherein a wireless communication between the wireless intraoral scanner (10) and the wireless transmission/reception device (80) is performed through a Wi-Fi communication network.
